# EUROPÄISCHE PATENTANMELDUNG

(11) **EP 2 711 033 A1**
(43) Veröffentlichungstag der Anmeldung: **26.03.2014**
(21) Anmeldenummer: 12006687.3
(22) Anmeldetag: 25.09.2012
(51) Int. Cl.: A61M 1/00, A61F 13/00

(54) **System für die Wundtherapie**

(71) Anmelder: Paul Hartmann AG, 89522 Heidenheim (DE)
(72) Erfinder: Smola, Hans, Prof. Dr., 66121 Saarbrücken (DE)
(74) Vertreter: Ter Meer Steinmeister & Partner

(57) **Zusammenfassung**

Die Erfindung betrifft allgemein die Wundtherapie und insbesondere Vorrichtungen bzw. Systeme für die Wundtherapie, wobei die Wundtherapie einen ersten Therapieabschnitt umfasst, in welchem eine Unterdrucktherapie durchgeführt wird, und einen darauf folgenden zweiten Therapieabschnitt, in welchem Wundtherapie unter Einsatz einer Wundauflage ohne Erzeugung eines Unterdrucks durchgeführt wird. Bevorzugt umfasst der erste Therapieabschnitt lediglich die inflammatorische Phase der Wundheilung. Die Wundauflage umfasst einen Saugkörper, der ein quellfähiges Polymer, insbesondere ein superabsorbierendes Polymer, umfasst.

## Beschreibung

Die Erfindung betrifft allgemein die Wundtherapie und insbesondere Vorrichtungen bzw. Systeme für die Wundtherapie

Unterdrucktherapie von Wunden, im Fachbereich auch bezeichnet als topische negative Druckbehandlung (TNP = topical negative pressure), NPWT (negative pressure wound therapy) oder Vakuum-assistierte Verschlussbehandlung (VAC = vacuum assisted closure), ist seit langem bekannt, findet aber insbesondere seit Mitte/Ende der 1990er Jahre in zunehmendem Maße Anwendung. Dabei wird üblicherweise ein Wundfüllmaterial in die Wunde eingelegt, das Wundgebiet mit einer Folie abgedeckt und mittels eines Drainageschlauchs und einer Vakuumpumpe ein Unterdruck im Wundraum erzeugt. Durch die Verwendung des Wundfüllmaterials wird der Druck gleichmäßig über die Wunde verteilt. Der Unterdruck bewirkt eine effektive Wundreinigung durch Abtransport von Wundexsudat. Als weitere Vorteile werden Förderung der Bildung von Granulationsgewebe und Verminderung der Wundödembildung genannt. Wie der Name VAC bereits suggeriert, wird die Unterdrucktherapie üblicherweise bis zum Wundverschluß durchgeführt. Der Wundverschluß gilt im Allgemeinen als erreicht, wenn über der vormaligen Wundstelle eine Schicht Epidermis vorhanden ist. Ein Wundverschluß kann durch Heilungsprozesse oder durch chirurgische Eingriffe erreicht werden.

Unterdrucktherapie wird insbesondere eingesetzt bei akuten oder chronischen Wunden, beispielsweise bei Geschwüren (z.B. Druckgeschwüren, diabetischen, neuropathischen oder venös bedingten Geschwüren), traumatischen Wunden, therapieresistenten Wunden, infizierten Wunden, postoperativen Wunden, sondierten Fisteln sowie Hautlappen und Hauttransplantaten. Chronische Wunden sind durch eine verlangsamte oder fehlende Wundheilung gekennzeichnet.

Vorrichtungen zur Unterdrucktherapie von Wunden sind entsprechend im Stand der Technik bekannt. So beschreibt beispielsweise die WO 1993/009727 A1 eine Vorrichtung zur Förderung der Wundheilung durch die Applikation von Unterdruck auf dem die Wunde aufweisenden und die Wunde umgebenden Hautbereich. Die Vorrichtung gemäß WO 1993/009727 A1 umfasst eine Vakuumeinrichtung zur Erzeugung des Unterdrucks, eine luftdichte Abdeckung der Wunde, welche mit der Vakuumeinrichtung in einer funktionellen Verbindung steht, sowie eine Wundauflage zur Positionierung an der Wunde innerhalb der luftdichten Abdeckung. Bei der Wundauflage handelt es sich vorzugsweise um einen offenzelligen Polymer-Schaumstoff, beispielsweise um Polyester-Schaum. Neben der Verwendung von offenzelligem Polymer-Schaumstoff wurden andere Materialien zur Herstellung von Wundauflagen für die Unterdrucktherapie von Wunden beschrieben.

Geräte zur Unterdrucktherapie von Wunden sind auch kommerziell erhältlich und umfassen kleine, tragbare Geräte, die den Patienten eine gewisse Mobilität ermöglichen bis hin zu stationär zu verwendenden Geräten, etwa in Langzeitpflegeeinrichtungen.

Als Nachteil der Unterdrucktherapie von Wunden werden in erster Linie der erhöhte apparative Aufwand und damit verbunden erhöhte Kosten ins Feld geführt sowie eine Beeinträchtigung des Patienten, insbesondere eine Beeinträchtigung der Mobilität des Patienten. Ferner wird die Unterdrucktherapie von Patienten häufig als schmerzhafter empfunden.

Der vorliegenden Erfindung liegt die Aufgabe zugrunde, eine Wundtherapie bereit zu stellen, welche den physiologischen Zustand der Wunde positiv beeinflusst. Ferner soll eine Wundtherapie bereit gestellt werden, welche den Patienten möglichst wenig beeinträchtigt. Zudem war es eine Aufgabe, die Wundtherapie hinsichtlich ihrer Kosten zu verbessern, ohne jedoch die klinische Effizienz der Wundtherapie zu beeinträchtigen oder aber dabei die klinische Effizienz sogar zu erhöhen, bzw. Systeme bereitzustellen, welche eine derart verbesserte Wundtherapie ermöglichen.

Es wurde überraschend gefunden, dass eine Kombination aus Unterdrucktherapie und darauf folgender Therapie in bevorzugt feuchtem bzw. feuchtnassem Medium ohne Einsatz von Unterdruck nicht nur die Kosten senkt, sondern auch die therapeutischen Ergebnisse verbessert. Insbesondere wurde unerwartet entdeckt, dass nach gleicher Gesamtbehandlungszeit die Bildung von Granulationsgewebe im Vergleich zu Unterdrucktherapie alleine verbessert ist.

Auch konnte die Beeinträchtigung des Patienten im Vergleich zur herkömmlichen Unterdrucktherapie verbessert werden, insbesondere im Hinblick auf das Schmerzempfinden und die Mobilität des Patienten.

Entsprechend umfasst die Erfindung gemäß einem ersten Aspekt eine Vorrichtung zur Durchführung von Wundtherapie mittels Unterdruck, wobei die Wundtherapie einen ersten Therapieabschnitt umfasst, in welchem Unterdrucktherapie mittels der Vorrichtung durchgeführt wird, und einen darauf folgenden zweiten Therapieabschnitt umfasst, in welchem Wundtherapie unter Einsatz einer Wundauflage ohne Erzeugung eines Unterdrucks durchgeführt wird, wobei die Wundauflage bevorzugt einen Saugkörper umfasst, welcher ein quellfähiges Polymer, insbesondere ein superabsorbierendes Polymer, umfasst. Anders ausgedrückt, gemäß diesem ersten Aspekt betrifft die Erfindung ein Erzeugnis zur Anwendung in der therapeutischen Behandlung von Wunden am menschlichen oder tierischen Körper, wobei das Erzeugnis eine Vorrichtung zur Unterdrucktherapie von Wunden umfasst, **dadurch gekennzeichnet, dass** die therapeutische Behandlung einen ersten Therapieabschnitt umfasst, in welchem die Unterdrucktherapie mittels der Vorrichtung durchgeführt wird, und einen darauf folgenden zweiten Therapieabschnitt umfasst, in welchem die therapeutische Behandlung unter Einsatz einer Wundauflage ohne Erzeugung eines Unterdrucks durchgeführt wird, wobei die Wundauflage bevorzugt einen Saugkörper umfasst, welcher ein quellfähiges Polymer, insbesondere ein superabsorbierendes Polymer, umfasst.

Die Vorrichtung zur Durchführung von Wundtherapie mittels Unterdruck wird der Einfachheit halber im Folgenden auch als Unterdrucktherapievorrichtung bezeichnet.

Die vorliegende Erfindung umfasst ferner die Verwendung einer Vorrichtung zur Unterdrucktherapie von Wunden in der therapeutischen Behandlung von Wunden am menschlichen oder tierischen Körper, **dadurch gekennzeichnet, dass** die therapeutische Behandlung einen ersten Therapieabschnitt umfasst, in welchem die Unterdrucktherapie mittels der Vorrichtung durchgeführt wird, und dass die therapeutische Behandlung einen darauf folgenden zweiten Therapieabschnitt umfasst, welcher unter Einsatz einer Wundauflage ohne Erzeugung eines Unterdrucks durchgeführt wird, wobei im zweiten Therapieabschnitt eine Wundauflage eingesetzt wird, welche einen Saugkörper enthält, der bevorzugt ein quellfähiges Polymer, insbesondere ein superabsorbierendes Polymer, umfasst.

Die Erfindung stellt gemäß einem zweiten Aspekt eine Wundauflage zur Wundtherapie - bevorzugt in feuchtem oder feuchtnassem Milieu - bereit, wobei die Wundtherapie einen ersten Therapieabschnitt umfasst, in welchem Wundtherapie unter Erzeugung von Unterdruck durchgeführt wird, und einen darauf folgenden zweiten Therapieabschnitt umfasst, in welchem Wundtherapie bevorzugt in feuchtem oder feuchtnassem Milieu unter Einsatz der Wundauflage ohne Erzeugung eines Unterdrucks durchgeführt wird. Insbesondere betrifft die Erfindung eine Wundauflage zur Anwendung in der therapeutischen Behandlung von Wunden am menschlichen oder tierischen Körper, wobei die Wundauflage bevorzugt einen Saugkörper umfasst, der ein quellfähiges Polymer, insbesondere ein superabsorbierendes Polymer, umfasst, **dadurch gekennzeichnet, dass** die therapeutische Behandlung einen ersten Therapieabschnitt umfasst, in welchem eine Wundtherapie mittels Unterdruck durchgeführt wird, und einen darauf folgenden zweiten Therapieabschnitt umfasst, in welchem die therapeutische Behandlung unter Einsatz der Wundauflage ohne Erzeugung eines Unterdrucks durchgeführt wird.

Anders ausgedrückt, gemäß dem zweiten Aspekt der Erfindung betrifft diese ein Erzeugnis zur Anwendung in der therapeutischen Behandlung von Wunden am menschlichen oder tierischen Körper, wobei das Erzeugnis eine Wundauflage umfasst, und wobei die Wundauflage bevorzugt einen Saugkörper umfasst, der ein quellfähiges Polymer, insbesondere ein superabsorbierendes Polymer, umfasst, **dadurch gekennzeichnet, dass** die therapeutische Behandlung einen ersten Therapieabschnitt umfasst, in welchem Unterdrucktherapie mittels einer Vorrichtung zur Unterdrucktherapie von Wunden durchgeführt wird, und einen darauf folgenden zweiten Therapieabschnitt umfasst, in welchem die therapeutische Behandlung unter Einsatz der Wundauflage ohne Erzeugung eines Unterdrucks durchgeführt wird.

Die vorliegende Erfindung umfasst ferner die Verwendung einer Wundauflage in der therapeutischen Behandlung von Wunden am menschlichen oder tierischen Körper, wobei die Wundauflage bevorzugt einen Saugkörper umfasst, der ein quellfähiges Polymer, insbesondere ein superabsorbierendes Polymer, umfasst, **dadurch gekennzeichnet, dass** die therapeutische Behandlung einen ersten Therapieabschnitt umfasst, in welchem Unterdrucktherapie mittels einer Vorrichtung zur Unterdrucktherapie von Wunden durchgeführt wird, und dass die therapeutische Behandlung einen darauf folgenden zweiten Therapieabschnitt umfasst, welcher unter Einsatz der Wundauflage ohne Erzeugung eines Unterdrucks durchgeführt wird.

Gemäß einem dritten Aspekt stellt die Erfindung eine Kombination aus Vorrichtung zur Durchführung von Wundtherapie mittels Unterdruck und Wundauflage zur Wundtherapie - bevorzugt in feuchtem oder feuchtnassem Milieu - bereit, wobei die Wundtherapie einen ersten Therapieabschnitt umfasst, in welchem Wundtherapie unter Einsatz der Vorrichtung unter Erzeugung von Unterdruck durchgeführt wird, und einen darauf folgenden zweiten Therapieabschnitt umfasst, in welchem Wundtherapie bevorzugt in feuchtem oder feuchtnassem Milieu unter Einsatz der Wundauflage ohne Erzeugung eines Unterdrucks durchgeführt wird. Anders ausgedrückt, gemäß dem dritten Aspekt der Erfindung betrifft diese ein Erzeugnis zur Anwendung in der therapeutischen Behandlung von Wunden am menschlichen oder tierischen Körper, wobei das Erzeugnis eine Vorrichtung zur Unterdrucktherapie von Wunden und eine Wundauflage umfasst, und wobei die Wundauflage bevorzugt einen Saugkörper umfasst, der ein quellfähiges Polymer, insbesondere ein superabsorbierendes Polymer, umfasst, **dadurch gekennzeichnet, dass** die therapeutische Behandlung einen ersten Therapieabschnitt umfasst, in welchem Unterdrucktherapie mittels einer Vorrichtung zur Unterdrucktherapie von Wunden durchgeführt wird, und einen darauf folgenden zweiten Therapieabschnitt umfasst, in welchem die therapeutische Behandlung unter Einsatz der Wundauflage ohne Erzeugung eines Unterdrucks durchgeführt wird.

Die vorliegende Erfindung umfasst ferner die Verwendung einer Kombination aus einer Vorrichtung zur Unterdrucktherapie von Wunden und einer Wundauflage, in der therapeutischen Behandlung von Wunden am menschlichen oder tierischen Körper, wobei die Wundauflage bevorzugt einen Saugkörper umfasst, der ein quellfähiges Polymer, insbesondere ein superabsorbierendes Polymer, umfasst, **dadurch gekennzeichnet, dass** die therapeutische Behandlung einen ersten Therapieabschnitt umfasst, in welchem Unterdrucktherapie mittels der Vorrichtung zur Unterdrucktherapie von Wunden durchgeführt wird, und einen darauf folgenden zweiten Therapieabschnitt umfasst, in welchem die therapeutische Behandlung unter Einsatz der Wundauflage ohne Erzeugung eines Unterdrucks durchgeführt wird.

Ein weiterer Gegenstand der Erfindung ist ein Verfahren zur therapeutischen Behandlung von Wunden am menschlichen oder tierischen Körper, umfassend
i) einen ersten Therapieabschnitt, in welchem Unterdrucktherapie mittels einer Vorrichtung zur Unterdrucktherapie von Wunden durchgeführt wird,
   wobei die Vorrichtung zur Durchführung von Wundtherapie mittels Unterdruck
   (a) ein luftundurchlässiges Abdeckmaterial zum luftdichten Verschließen von Wunde und Wundumgebung, wobei ein Wundraum gebildet wird,
   (b) ein Mittel zum Bereitstellen von Fluidkommunikation zwischen dem Wundraum und einer außerhalb des Abdeckmaterials befindlichen Unterdruckquelle, so dass ein Unterdruck im Wundraum herstellbar ist und Flüssigkeiten aus dem Wundraum absaugbar sind,
   (c) einer ersten Wundauflage, welche eine im Wesentlichen gleichmäßige Verteilung des Unterdrucks im Wundraum ermöglicht
   umfasst,
ii) einen darauf folgenden zweiten Therapieabschnitt, in welchem die therapeutische Behandlung (bevorzugt in feuchtem oder feuchtnassem Milieu) unter Einsatz einer zweiten Wundauflage ohne Erzeugung eines Unterdrucks durchgeführt wird,
wobei die zweite Wundauflage bevorzugt einen Saugkörper umfasst, der ein quellfähiges Polymer, insbesondere ein superabsorbierendes Polymer, umfasst.

Bei der ersten Wundauflage handelt es sich bevorzugt um eine Wundauflage, welche einen offenzelligen Schaumstoff, beispielsweise einen offenzelligen Polyurethanschaumstoff, umfasst. Gemäß einer alternativen Ausführungsform kann jedoch auch für den ersten Therapieabschnitt als erste Wundauflage ein Saugkörper verwendet werden, welcher ein quellfähiges Polymer, insbesondere ein superabsorbierendes Polymer, umfasst.

Gegenstand der Erfindung ist insbesondere auch ein Verfahren zur therapeutischen Behandlung von Wunden am menschlichen oder tierischen Körper, umfassend
i) einen ersten Therapieabschnitt, in welchem Unterdrucktherapie mittels einer Vorrichtung zur Unterdrucktherapie von Wunden zur Entzündungsbekämpfung und/oder Ödemmobilisierung im Wundbereich durchgeführt wird, wobei die Vorrichtung zur Durchführung von Wundtherapie mittels Unterdruck
   (a) ein luftundurchlässiges Abdeckmaterial zum luftdichten Verschließen von Wunde und Wundumgebung, wobei ein Wundraum gebildet wird,
   (b) ein Mittel zum Bereitstellen von Fluidkommunikation zwischen dem Wundraum und einer außerhalb des Abdeckmaterials befindlichen Unterdruckquelle, so dass ein Unterdruck im Wundraum herstellbar ist und Flüssigkeiten aus dem Wundraum absaugbar sind,
   (c) einer ersten Wundauflage, welche eine im Wesentlichen gleichmäßige Verteilung des Unterdrucks im Wundraum ermöglicht
   umfasst,
ii) einen darauf folgenden zweiten Therapieabschnitt, in welchem unter Einsatz einer zweiten Wundauflage eine verstärkte Stimulation der Bildung von Granulationsgewebe im Wundbereich
   oder
   eine verstärkte Stimulation der Gefäßneubildung im Wundbereich
   oder
   eine Erhöhung der Fibroblastendichte im oberflächlichen Granulationsgewebe einer Wunde,
   jeweils ohne Erzeugung eines Unterdrucks, durchgeführt wird,
   wobei die zweite Wundauflage bevorzugt einen Saugkörper umfasst, der ein quellfähiges Polymer, insbesondere ein superabsorbierendes Polymer, umfasst.

Schließlich ist Gegenstand der Erfindung eine Kombination aus Vorrichtung zur Unterdrucktherapie von Wunden und einer Wundauflage, zur Anwendung in der therapeutischen Behandlung von Wunden am menschlichen oder tierischen Körper, wobei die Wundauflage bevorzugt einen Saugkörper umfasst, der ein quellfähiges Polymer, insbesondere ein superabsorbierendes Polymer, umfasst, **dadurch gekennzeichnet, dass** die therapeutische Behandlung einen ersten Therapieabschnitt umfasst, in welchem Unterdrucktherapie mittels der Vorrichtung durchgeführt wird, und einen darauf folgenden zweiten Therapieabschnitt umfasst, in welchem die therapeutische Behandlung unter Einsatz der Wundauflage ohne Erzeugung eines Unterdrucks durchgeführt wird.

Es wurde überraschend gefunden, dass die Dauer der Unterdrucktherapie signifikant abgekürzt werden und durch Wundtherapie unter Verwendung der erfindungsgemäßen Wundauflage, d.h. bevorzugt in feuchtem oder feuchtnassem Milieu, ohne Anwendung von Unterdruck ersetzt werden kann, und dabei wider Erwarten die Bildung von Granulationsgewebe nicht leidet, sondern sogar verbessert wird. Neben der Kostenersparnis und verbesserten Patienten-Compliance wurden sogar verbesserte klinische Ergebnisse erzielt.

Insbesondere wurde unerwarteter Weise gefunden, dass durch das erfindungsgemäße Therapieschema
- verstärkte Stimulation der Bildung von Granulationsgewebe im Wundbereich, über das bei der Unterdrucktherapie normalerweise erreichbare Maß hinaus erreicht wird;
- eine verstärkte Stimulation der Gefäßneubildung im Wundbereich erreicht wird; und/oder
- eine deutliche Erhöhung der Fibroblastendichte im oberflächlichen Granulationsgewebe einer Wunde erreicht wird.

Folglich ist Gegenstand der Erfindung eine Wundauflage, zur Anwendung in der therapeutischen Behandlung von Wunden am menschlichen oder tierischen Körper, wobei die Wundauflage bevorzugt einen Saugkörper umfasst, der ein quellfähiges Polymer, insbesondere ein superabsorbierendes Polymer, umfasst, **dadurch gekennzeichnet, dass** die Wundauflage beim Abklingen der inflammatorischen Phase nach einer vorangegangenen Unterdrucktherapie eingesetzt wird.

Weiterhin ist Gegenstand der Erfindung eine Wundauflage, zur Anwendung in der therapeutischen Behandlung von Wunden am menschlichen oder tierischen Körper, wobei die Wundauflage bevorzugt einen Saugkörper umfasst, der ein quellfähiges Polymer, insbesondere ein superabsorbierendes Polymer, umfasst, **dadurch gekennzeichnet, dass** die Wundauflage in einer zweiten Therapiephase nach einer auf Entzündungsbekämpfung und/oder Ödemmobilisierung zielenden ersten Therapiephase, eingesetzt wird, wobei die erste Therapiephase eine Unterdrucktherapie mittels einer Vorrichtung zur Unterdrucktherapie von Wunden umfasst und wobei die zweite Therapiephase unter Einsatz der Wundauflage ohne Erzeugung eines Unterdrucks durchgeführt wird. Die Ausdrücke "Therapieabschnitt" und "Therapiephase" werden hier synonym verwendet.

Weiterhin ist Gegenstand der Erfindung eine Wundauflage, zur Anwendung in der therapeutischen Behandlung von Wunden am menschlichen oder tierischen Körper, wobei die Wundauflage bevorzugt einen Saugkörper umfasst, der ein quellfähiges Polymer, insbesondere ein superabsorbierendes Polymer, umfasst, **dadurch gekennzeichnet, dass** die Wundauflage zur verstärkten Stimulation der Bildung von Granulationsgewebe im Wundbereich nach einer vorangegangenen Unterdrucktherapie eingesetzt wird.

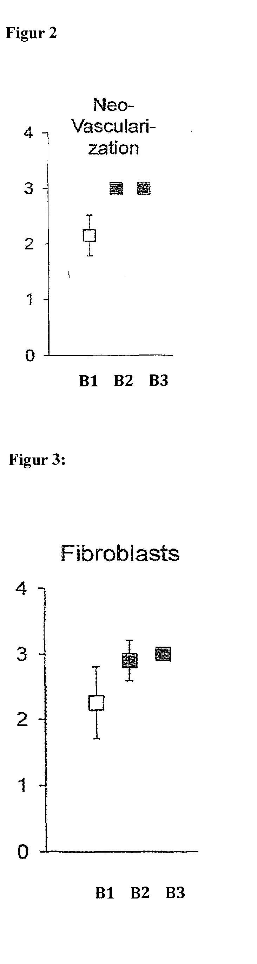

Weiterhin ist Gegenstand der Erfindung eine Wundauflage, zur Anwendung in der therapeutischen Behandlung von Wunden am menschlichen oder tierischen Körper, wobei die Wundauflage bevorzugt einen Saugkörper umfasst, der ein quellfähiges Polymer, insbesondere ein superabsorbierendes Polymer, umfasst, **dadurch gekennzeichnet, dass** die Wundauflage zur verstärkten Stimulation der Gefäßneubildung im Wundbereich nach einer vorangegangenen Unterdrucktherapie eingesetzt wird. Zur vorteilhaften Stimulation der Gefäßneubildung wird auch auf nachstehend erläuterte Figur 2 verwiesen.

Weiterhin ist Gegenstand der Erfindung eine Wundauflage, zur Anwendung in der therapeutischen Behandlung von Wunden am menschlichen oder tierischen Körper, wobei die Wundauflage bevorzugt einen Saugkörper umfasst, der ein quellfähiges Polymer, insbesondere ein superabsorbierendes Polymer, umfasst, **dadurch gekennzeichnet, dass** die Wundauflage zur Erhöhung der Fibroblastendichte im oberflächlichen Granulationsgewebe einer Wunde nach einer vorangegangenen Unterdrucktherapie eingesetzt wird. Zur Erhöhung der Fibroblastenbildung wird auch auf nachstehend erläuterte Figur 3 verwiesen.

In der Literatur werden drei wesentliche Heilungsphasen einer Wunde, insbesondere bei Wunden mit Gewebeverlust, beschrieben. Hierzu gehört die Entzündungs- (inflammatorische) oder exsudative Phase zur Blutstillung und Wundreinigung (Phase 1, Reinigungsphase), die proliferative Phase zum Aufbau von Granulationsgewebe (Phase 2, Granulationsphase) und die Differenzierungsphase zur Epithelisierung und Narbenbildung (Phase 3, Epithelisierungsphase).

Die inflammatorische Phase tritt üblicherweise direkt nach der Wundbildung auf. Sie ist gekennzeichnet durch Gefäßkonstriktion, Aktivierung der Gerinnungskaskade und komplexe immunologische Abläufe. Durch die Freisetzung chemoattraktiver Substanzen (z.B. Chemokine) kann eine lokale Entzündungsreaktion hervorgerufen werden. Es gibt vier wesentliche Entzündungszeichen: Ödem, Exsudation, Rötung und Schmerz. Die umliegenden Gefäße können sich erweitern, und Entzündungszellen wandern in den Entzündungsortein. Diese können Mikroorganismen und Gewebsnekrosen beseitigen. Dadurch kann eine Reinigung der Wunde erfolgen.

Bei der darauf folgenden Proliferations- oder Granulationsphase erfolgt der Aufbau von neuem Gewebe mit Gefäßeinsprossung und Defektauffüllung durch Granulationsgewebe. Dies ist die Grundvoraussetzung für die spätere Epithelisierung. Fibroblasten aus dem umliegenden Gewebe können in das Fibrinnetz migrieren und es als provisorische Matrix nutzen. Es beginnt der Aufbau von Kollagenfasern. Das provisorische Fibringerüst wird durch das Granulationsgewebe ersetzt. Die gleichzeitig stattfindende Angiogenese versorgt das Granulationsgewebe mit einsprossenden Kapillaren und Gefäßen.

Mit der Differenzierungs- oder Umbauphase beginnt üblicherweise die Ausreifung der kollagenen Fasern. Die Wunde kontrahiert sich durch die Umwandlung von Fibroblasten in Fibrozyten sowie Myofibroblasten. Dadurch schrumpft das Narbengewebe und es führt zu einer Verkleinerung der Wunde. Die Epithelisierung vom Wundrand her bringt die Wundheilung zum Abschluss.

Der erste Therapieabschnitt umfasst bevorzugt lediglich die inflammatorische Phase bzw. einen Teil der inflammatorischen Phase. Anders ausgedrückt erstreckt sich der erste Therapieabschnitt bevorzugt bis zum Abklingen oder Abschluss der inflammatorischen Phase. Das Abklingen der inflammatorischen Phase ist für den Arzt erkennbar am Abklingen eines oder mehrerer der Entzündungssymptome: Ödem, Exsudation, Rötung und Schmerz. Bevorzugt wird der erste Therapieabschnitt beendet, bevor eine sichtbare Granulation erkennbar ist.

Die Dauer des ersten Therapieabschnitts hängt von den individuellen Gegebenheiten ab. Erfolge wurden jedoch bereits erzielt, wenn der erste Therapieabschnitt sich beispielsweise über einen Zeitraum von nur 1, 2, 3, 4, 5, 6, 7 oder 8 Tagen erstreckt, z.B. zwischen 3 und 7 oder 4 und 6 Tagen, insbesondere 4 Tage. Diese Zeitspanne ist kürzer als die typische Dauer einer Unterdrucktherapie. Die Unterdrucktherapie braucht insbesondere nicht mehr bis zum Wundverschluß durchgeführt zu werden.

Der zweite Therapieabschnitt ist ebenfalls den individuellen Gegebenheiten anzupassen, kann sich aber beispielsweise vorteilhaft über einen Zeitraum von 1 bis 1000 Tage, bevorzugt von zwei bis einhundert Tage, z.B. drei bis zwanzig Tage, insbesondere vier bis zehn Tage erstrecken.

Experimentell hat sich als vorteilhaft herausgestellt eine Kombination von einem ersten Therapieabschnitt von vier Tagen und einem zweiten Therapieabschnitt von ebenfalls vier Tagen. Wie später anhand von Figuren gezeigt wird, bringt diese Alternative zu einer herkömmlichen 8-tägigen Unterdrucktherapie bessere Wundtherapieergebnisse.

Bevorzugt folgt der zweite Therapieabschnitt unmittelbar auf den ersten Therapieabschnitt.

Die erfindungsgemäß zu verwendende Vorrichtung zur Durchführung von Wundtherapie mittels Unterdruck kann eine aus dem Stand der Technik bekannte Vorrichtung sein. Vorteilhaft umfasst die Vorrichtung zur Durchführung der Unterdrucktherapie
(a) ein luftundurchlässiges Abdeckmaterial zum luftdichten Verschließen von Wunde und Wundumgebung, wobei ein Wundraum gebildet wird,
(b) ein Mittel zum Bereitstellen von Fluidkommunikation zwischen dem Wundraum und einer außerhalb des Abdeckmaterials befindlichen Unterdruckquelle, so dass ein Unterdruck im Wundraum herstellbar ist und Flüssigkeiten aus dem Wundraum absaugbar sind,
(c) eine Wundauflage, die bevorzugt einen offenzelligen Schaumstoff umfasst.

Die erfindungsgemäß einsetzbare Unterdrucktherapievorrichtung umfasst ein Abdeckmaterial (a) zum luftdichten Verschließen der Wunde. Unter "luftdichten Verschließen" soll hier nicht verstanden werden, dass keinerlei Gasaustausch zwischen dem Wundraum und seiner Umgebung stattfindet. Vielmehr ist mit "luftdichten Verschließen" in diesem Zusammenhang gemeint, dass der für die Unterdrucktherapie nötige Unterdruck aufrechterhalten werden kann. Es können deshalb auch Abdeckmaterialien eingesetzt werden, welche eine geringfügige Gas-Permeabilität aufweisen, so lange der für die Unterdrucktherapie notwendige Unterdruck aufrechterhalten werden kann.

Das luftdichte Abdeckmaterial kann beispielsweise in Form einer aus einem festen Material bestehenden Schale oder in Form einer flexiblen Folie vorliegen. Vorstellbar sind auch Kombinationen dieser. In einer bevorzugten Ausführungsform der Erfindung umfasst das Abdeckmaterial zum luftdichten Verschließen der Wunde ein wasserunlösliches Polymer, oder eine Metallfolie. Das Abdeckmaterial weist bevorzugt eine Dicke von 10 µm bis 10.000 µm, insbesondere von 25 µm bis 100 µm auf.

In einer bevorzugten Ausführungsform der Erfindung handelt es sich bei dem Abdeckmaterial (a) um ein wasserunlösliches Polymer. Bevorzugt weist das wasserunlösliche Polymer eine Löslichkeit von 10 mg/l oder weniger, mehr bevorzugt von 1 mg/ml oder weniger, insbesondere von 0,0001 bis 1 mg/ml auf (bestimmt gemäß Säulenelutionsmethode nach EU-Richtlinie RL67-548-EWG, Anhang V Kap. A6). Beispiele sind Polyurethan, Polyester, Polypropylen, Polyethylen, Polyamid oder Polyvinylchlorid, Polyorganosiloxan (Silikon), oder eine Mischung daraus. Die genannten Polymere liegen hierbei bevorzugt in nicht zellulärer Form vor. In einer bevorzugten Ausführungsform weist das Abdeckungsmaterial einen Wasserdampfdurchlässigkeit von 100 bis 2500 g/m² x 24h, mehr bevorzugt von 500 bis 2000 g/m² x 24h, noch mehr bevorzugt von 800 bis 1600 g/m² x 24h, insbesondere von 1050 bis 1450 g/m² x 24h, bestimmt gemäß DIN EN 13726-2 bei 23 °C und 85 % relativer Luftfeuchte, auf.

Als Abdeckmaterial können auch Fertigprodukte eingesetzt werden, welche die vorstehend genannten Eigenschaften aufweisen. Als geeignetes Abdeckmaterial für die erfindungsgemäß zu verwendende Unterdrucktherapievorrichtung hat sich der Polyurethanfilm der Marke Hydrofilm^{®} (Paul Hartmann AG, Deutschland) oder Visulin^{®} (Paul Hartmann AG, Deutschland) erwiesen.

Das Abdeckmaterial wird üblicherweise in der Wundumgebung oder am Wundrand so befestigt, dass ein luftdichter Wundverschluss gewährleistet ist. Dabei kann es zweckmäßig sein, wenn das Abdeckmaterial vollflächig selbstklebend ausgestattet ist oder einen selbstklebenden Rand aufweist. Alternativ können Befestigung und Abdichtung beispielsweise mit einer Klebefolie, mit einem flüssigen Kleber oder mit einer Abdichtmasse erfolgen. Möglich ist jedoch auch, dass das Abdeckmaterial lediglich durch den während der Unterdruckbehandlung erzeugten Unterdruck an der Wunde gehalten wird.

Die erfindungsgemäße Vorrichtung zur Unterdrucktherapie umfasst ein Mittel (b) zum Bereitstellen von Fluidkommunikation zwischen dem Wundraum und einer außerhalb des Abdeckmaterials befindlichen Unterdruckquelle, so dass ein Unterdruck im Wundraum herstellbar ist und Flüssigkeit aus dem Wundraum absaugbar ist.

Der Ausdruck "Unterdruck im Wundraum" bezeichnet im Zusammenhang mit der Erfindung einen innerhalb des Wundverbandes gegenüber dem Umgebungsluftdruck (atmosphärischer Luftdruck) erniedrigten Luftdruck. Unter "innerhalb des Wundverbandes" wird der durch das Abdeckmaterial und die Wunde gebildete Zwischenraum verstanden. "Unterdruck" wird häufig auch als "negativer Druck" bezeichnet.

Die Fluidkommunikation kann beispielsweise mit einer Verbindungsleitung und/oder mit einem Unterdruck-Anschlussstück hergestellt werden. Unterdruck-Anschlussstücke sind dem Fachmann auch unter der Bezeichnung "Port" bekannt.

Bei einer Ausführungsform handelt es sich bei dem Mittel zur funktionellen Verbindung des Wundraums mit einer außerhalb des Abdeckmaterials befindlichen Unterdruckquelle um mindestens eine Verbindungsleitung. Die mindestens eine Verbindungsleitung kann durch das Abdeckmaterial hindurchgeführt werden. Alternativ kann die mindestens eine Verbindungsleitung unter dem Rand des Abdeckmaterials durchgeführt werden. In beiden Fällen ist die Durchtrittsstelle luftdicht abzudichten, so dass der gewünschte Unterdruck im Verband aufrecht erhalten werden kann. Als Abdichtmittel eignet sich beispielsweise eine Klebefolie, eine Klebemasse, oder ein Klebestreifen.

Bei der Verbindungsleitung kann es sich beispielsweise um einen Schlauch, um ein Rohr oder um einen anderen Körper mit einem Hohlraum handeln. Ein geeigneter Schlauch ist beispielsweise ein Silicon-Drainageschlauch.

In einer weiteren Ausführungsform handelt es sich bei dem Mittel zur funktionellen Verbindung des Wundraums mit einer außerhalb des Abdeckmaterials befindlichen Unterdruckquelle um ein Unterdruck-Anschlussstück (Port), welches auf einer der Innen- oder Außenseiten des Abdeckmaterials befestigt werden kann, wobei das Abdeckmaterial geeignete Öffnungen aufweist. Auch bei dieser Ausführungsform ist auf eine luftdichte Abdichtung entweder der Durchtrittsöffnung (Port innen) oder der Auflagefläche (Port außen) zu achten. Die Abdichtung kann beispielsweise mit einer Klebefolie, mit einer Klebemasse, oder mit einem Klebestreifen hergestellt werden. Es ist auch denkbar, dass der Port selbst über entsprechende Befestigungseinrichtungen, wie beispielsweise Klebeflächen, verfügt.

In einer bevorzugten Ausführungsform werden das Abdeckmaterial (a) und das Mittel zum Bereitstellen von Fluidkommunikation zwischen dem Wundraum und einer außerhalb des Abdeckmaterials befindlichen Unterdruckquelle (b) bereits gebrauchsfertig miteinander verbunden zur Verfügung gestellt.

Neben den vorstehend beschriebenen Bestandteilen (a) und (b) umfasst die erfindungsgemäße Vorrichtung zur Unterdrucktherapie noch Bestandteil (c), die Wundauflage, die offenzelligen Schaumstoff umfasst oder aus diesem besteht.

Im Rahmen dieser Anmeldung bedeutet der Begriff offenzellig, dass im Schaumstoff (c) mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind.

Die Wundauflage (c) der Unterdruckvorrichtung ermöglicht beispielsweise auch eine im Wesentlichen gleichmäßige Verteilung des Unterdrucks im Wundraum.

In einer bevorzugten Ausführungsform ist der offenzellige Schaumstoff ein Polyurethanschaumstoff, der erhältlich ist durch Umsetzung einer Mischung, umfassend die Komponenten (i) Polyisocyanat, (ii) Polyol, bevorzugt Polyesterpolyol, (iii) Treibmittel, und (iv) Katalysator.

Als Polyisocyanate (i-PUR) können allgemein bekannte aliphatische, cycloaliphatische und/oder insbesondere aromatische Polyisocyanate eingesetzt werden. Zur Herstellung der Polyurethane eignen sich beispielsweise Diphenylmethandiisocyanat (MDI), hier insbesondere 4,4'- Diphenylmethandiisocyanat (4,4'-MDI), Mischungen aus monomeren Diphenylmethandiisocyanaten und höherkernigen Homologen des Diphenylmethandiisocyanats (PMDI), Tetramethylendiisocyanat (TMDI), Hexamethylendiisocyanat (HDI), Toluylendiisocyanat (TDI) oder Mischungen daraus.

Als gegenüber Isocyanaten reaktive Verbindungen (ii-PUR) werden üblicherweise Polyole wie Polyetherole und/oder Polyesterole verwendet. Bevorzugt werden Polyesterpolyole in der Komponente (ii-PUR) verwendet. Die verwendeten Polyesterole (ii-PUR) werden im allgemeinen durch Kondensation von mehrfunktionellen Alkoholen, vorzugsweise Diolen, mit 2 bis 12 Kohlenstoffatomen, vorzugsweise 2 bis 6 Kohlenstoffatomen, mit mehrfunktionellen Carbonsäuren mit 2 bis 12 Kohlenstoffatomen, bevorzugt 4 bis 8 Kohlenstoffatomen hergestellt. Beispiele für geeignete Säuren sind Bernsteinsäure, Glutarsäure, Adipinsäure, Phthalsäure, Isophthalsäure, und/oder Terephthalsäure und Gemische hiervon. Adipinsäure ist besonders bevorzugt. Beispiele für geeignete zwei- und mehrwertige Alkohole sind Ethandiol, Diethylenglykol, 1,4-Butandiol, 1,5-Pentandiol, und/oder 1,6-Hexandiol und Gemische hiervon. 1,4-Butandiol ist besonders bevorzugt. Die Verbindungen (ii-PUR) können in Mischung mit Kettenverlängerungs- und/oder Vernetzungsmitteln verwendet werden.

Als Katalysatoren (iv-PUR) können Verbindungen eingesetzt werden, welche die Reaktion der Komponente (i-PUR) mit der Komponente (ii-PUR) beschleunigen. In Frage kommen beispielsweise tertiäre Amine und/oder organische Metallverbindungen, insbesondere Zinnverbindungen. Beispielsweise können als Katalysatoren folgende Verbindungen eingesetzt werden: Triethylendiamin, Aminoalkyl- und/oder Aminophenylimidazole und/oder Zinn-(II)salze von organischen Carbonsäuren. Katalysatoren werden im allgemeinen in einer Menge von 0,1 bis 5 Gew.-% bezogen auf das Gewicht der Komponente (ii-PUR) eingesetzt.

Als Treibmittel (iii-PUR) können allgemein bekannte chemisch oder physikalisch wirkende Verbindungen eingesetzt werden. Als physikalisch wirkendes Treibmittel kann bevorzugt Wasser eingesetzt werden, welches durch Reaktion mit den Isocyanatgruppen Kohlendioxid bildet. Beispiele für physikalische Treibmittel sind (cyclo)aliphatische Kohlenwasserstoffe, vorzugsweise solche mit 4 bis 8, besonders bevorzugt 4 bis 6 und insbesondere 5 Kohlenstoffatomen, teilhalogenierte Kohlenwasserstoffe oder Ether, Ketone oder Acetate. Die Menge der eingesetzten Treibmittel richtet sich nach der angestrebten Dichte der Schaumstoffe. Die unterschiedlichen Treibmittel können einzeln oder in beliebigen Mischungen untereinander zum Einsatz kommen. Besonders bevorzugt wird nur Wasser als Treibmittel eingesetzt, im allgemeinen in einer Menge von 0,1 bis 5 Gew.-%, insbesondere von 2,5 bis 4 Gew.-%, bezogen auf das Gewicht der Komponente (ii-PUR). Physikalische Treibmittel werden bevorzugt in einer Menge von < 0,5 Gew.-%, bezogen auf das Gewicht der Komponente (ii-PUR), eingesetzt.

Die Umsetzung erfolgt gegebenenfalls in Anwesenheit von (v-PUR) Hilfs- und/oder Zusatzstoffen, wie z. B. Füllstoffen, Zellreglern, Zellöffnern, oberflächenaktiven Verbindungen und/oder Stabilisatoren gegen oxidativen, thermischen oder mikrobiellen Abbau oder Alterung.

Bevorzugt ist der offenzellige Polyurethanschaumstoff erhältlich durch Umsetzung einer Mischung, die die folgenden Komponenten umfasst oder aus diesen besteht:
(i) Polyisocyanat, ausgewählt aus MDI, PMDI, TDI und/oder HDI, (ii) Polyesterpolyol, wobei das Polyesterpolyol bevorzugt erhältlich ist durch Umsetzung einer Dicarbonsäure mit 4 bis 8 Kohlenstoffatomen mit einen Dialkohol mit 2 bis 6 Kohlenstoffatomen und/oder bevorzugt ein gewichtsmittleres Molekulargewicht von 500 bis 4000 g/mol aufweist, (iii) Treibmittel, und (iv) Katalysator.

Bevorzugt weist der offenzellige Schaumstoff, insbesondere Polyurethanschaumstoff, nach dreitägiger Lagerung in Rinderserum eine Zugfestigkeit zwischen 80 kPa und 300 kPa auf, gemessen gemäß DIN 53571.

Bevorzugt weist der offenzellige Schaumstoff, insbesondere Polyurethanschaumstoff, eine Luftdurchlässigkeit von 1000 bis 8000 1/(m²sec) auf, gemessen gemäß DIN EN ISO 9237.

Die Wundauflage (c) der Unterdruckvorrichtung kann Silber in Form von Silberionen oder in Form von atomarem Silber enthalten. Bevorzugt ist Silber in Form einer Silberbeschichtung auf der Wundauflage aufgebracht. Alternativ kann das Silber innerhalb der Wundauflage verteilt sein. Beispielsweise kann bei offenzelligen Schaumstoffen Silber bereits in die härtbare Zusammensetzung mit eingebracht werden. Bevorzugt enthält der Schaumstoff (c) 0,000001 bis 0,1 Gew.-%, mehr bevorzugt 0,0001 bis 0,01 Gew.-% Silber, bezogen auf das Gesamtgewicht des Schaumstoffs (c).

In einer bevorzugten Ausführungsform der Erfindung weist die Wundauflage (c), insbesondere der offenzellige Schaumstoff, eine Dicke von 1 bis 50 mm, insbesondere von 15 mm bis 30 mm auf.

In einer bevorzugten Ausführungsform der Erfindung umfasst die Vorrichtung zur Unterdrucktherapie von Wunden mindestens eine Wundkontaktschicht zum Einbringen zwischen Wundauflage (c) und Wundoberfläche. Die zusätzliche Wundkontaktschicht kann mit der Wundauflage (c) haftend oder nicht haftend verbunden sein. Als Wundkontaktschicht kommt grundsätzlich jede aus dem Stand der Technik bekannte Wundkontaktschicht in Frage, solange einerseits ein Durchtritt von Wundexsudat gewährleistet ist und das Material anderseits keine Neigung zum Verwachsen oder Verkleben mit dem Wundgewebe aufweist. Besonders geeignete Wundkontaktschichten sind Salbenkompressen der Marke Hydrotüll^{®}, Atrauman^{®} und Atrauman Ag^{®} (Paul Hartmann AG, Deutschland).

In einer weiteren Ausführungsform können auch Vorrichtungen zur Unterdrucktherapie von Wunden eingesetzt werden, die als Wundauflage (c) einen offenzelligen Schaumstoff auf Basis eines quervernetzten Polyorganosiloxans enthält. Ein Vorteil des offenzelligen Schaumstoffs auf Basis eines quervernetzten Polyorganosiloxans besteht darin, dass er eine weiche Wundauflage für die Unterdrucktherapie von Wunden bereitstellt und eine gleichmäßige Druckverteilung auf den Wundgrund gewährleistet. Durch die Bereitstellung einer weichen Wundauflage und durch die gleichmäßige Druckverteilung kann die Unterdruckbehandlung schonend und wirksam durchgeführt werden.

In einer weiteren Ausführungsform umfasst die Vorrichtung zur Unterdrucktherapie von Wunden mindestens eine zusätzliche Druckverteilungsschicht zwischen der Wundauflage (c) und dem Abdeckmaterial (a). Der Vorteil einer zusätzlichen Druckverteilungsschicht kann darin bestehen, dass der durch den Verband auf den Wundgrund ausgeübte Druck durch die Verwendung der Druckverteilungsschicht noch gleichmäßiger verteilbar ist. Weiterhin kann die Druckverteilungsschicht zusätzliches Wundexsudat speichern und/oder weiterleiten.

Die zusätzliche Druckverteilungsschicht kann aus einem offenzelligen oder halboffenzelligen Schaumstoff, einem Abstandsgewirke, aus einer Textilschicht, aus einem strukturiertem Gel, oder aus einer durchlässigen Nonwoven-Schicht bestehen. Geeignete Textilschichten sind unter anderem ES-Kompressen oder Gittertülls. Die zusätzliche Druckverteilungsschicht kann so ausgebildet sein, dass Flüssigkeit, wie Wundexsudat, durch sie hindurchgeleitet wird. Dazu kann die Druckverteilungsschicht geeignete Kanäle oder Öffnungen enthalten, oder aus einem für Flüssigkeiten durchlässigen Material bestehen.

Die vorstehenden Erläuterungen zu bevorzugten Ausführungsformen der Punkte (a) bis (c) der Vorrichtung zur Unterdrucktherapie beziehen sich auf alle Aspekte der vorliegenden Erfindung, d.h. auf das erfindungsgemäße Erzeugnis, auf die erfindungsgemäße Vorrichtung, auf die erfindungsgemäße Verwendung und das erfindungsgemäße Verfahren.

Die Wundauflage (c) der erfindungsgemäßen Unterdruckvorrichtung ("erste Wundauflage") ist grundsätzlich nicht zu verwechseln mit der erfindungsgemäßen Wundauflage zur Wundtherapie, die im zweiten Therapieabschnitt zum Einsatz kommt und im Folgenden näher beschrieben wird ("zweite Wundauflage"). Es wird allerdings angemerkt, dass die im folgenden beschriebenen Wundauflagen grundsätzlich auch als Wundauflagen (c) geeignet und entsprechend in der vorliegenden Erfindung umfasst sind.

Der zweite Therapieabschnitt wird im Allgemeinen "ohne Unterdruck" durchgeführt. Hierbei ist nicht zu verstehen, dass eine Wundbehandlung mit Unterdruckvorrichtung jedoch ohne angelegten Unterdruck durchgeführt wird. Vielmehr ist darunter zu verstehen, dass im zweiten Therapieabschnitt auf den Einsatz einer Unterdruckvorrichtung vollständig verzichtet wird.

Die Wundauflage ("zweite Wundauflage") ist im Allgemeinen eine Wundauflage, die eine Wundbehandlung im feuchten oder feuchtnassen Milieu erlaubt. Unter feucht oder feuchtnass wird hierbei verstanden, dass die Wunde unter der Wundauflage eine feuchte oder feuchtnasse Oberfläche aufweist, d.h. die Wunde trocknet nicht (zumindest nicht vollständig) unter der Oberfläche. Bevorzugt umfasst die Wundauflage ("zweite Wundauflage") einen Saugkörper, der ein quellfähiges Polymer, insbesondere ein superabsorbierendes Polymer, umfasst.

Gemäß einer besonders bevorzugten Ausführungsform umfasst die Wundauflage einen Saugkörper, der eine mindestens bereichsweise flüssigkeitsdurchlässige Hülle und darin angeordnet ein quellfähiges Polymer, insbesondere ein superabsorbierendes Polymer, umfasst. Anders ausgedrückt ist das quellfähige Polymer von der Hülle umgeben.

Die mindestens bereichsweise flüssigkeitsdurchlässige Hülle erlaubt über ihre gesamte Fläche oder zumindest in bestimmten Bereichen der Hülle den Durchtritt von Flüssigkeiten. Die Hülle kann aus einem einzigen Material bestehen oder mehrere Materialien umfassen. Zumindest auf einer im Gebrauch wundzugewandten Seite der Wundauflage kann die Hülle in vorteilhafter Weise ein textiles (Flächen-)Material, wie z.B. ein Gestrick, ein Gewirke oder Gewebe, insbesondere aus Polyolefin, insbesondere aus Polypropylen, sein bzw. umfassen. Die Hülle kann vorteilhaft mindestens teilweise aus einer textilen Fläche besteht, welche in Längs-, Quer-, und Diagonalrichtung nicht-elastisch dehnbar ist, wie in EP 0 594 034 offenbart.

Das quellfähige Polymer ist oder umfasst bevorzugt ein superabsorbierendes Polymer. Unter einem superabsorbierenden Polymer (SAP) wird allgemein ein wasserunlösliches, quellbares Polymer verstanden, welches ein Vielfaches seines Eigengewichtes an Flüssigkeit, zum Beispiel Wasser, aufnehmen kann. Die Flüssigkeitsaufnahme führt zur Ausbildung eines Hydrogels. Im Rahmen dieser Erfindung wird unter dem Ausdruck superabsorbierendes Polymer insbesondere ein Polymer verstanden, welches gemäß der Standard-Testmethode WSP 240.2 (05) einen w-Wert ("free swell capacity") von mindestens 10 g/g, vorzugsweise mindestens 20 g/g aufweist. Die Testmethode WSP 240.2 (05) zur Bestimmung des w-Wertes ist in "Standard Test Methods for the Nonwovens and Related Industries", Ausgabe 2008 beschrieben (herausgegeben von "EDANA, International Association Serving the Nonwovens and Related Industries", Cary, NC, USA und "INDA, Association of the Nonwovens Fabrics Industry", Brüssel, Belgien). WSP 240.2 (05) nach EDANA ist eine Standard-Testmethode zur Ermittlung des w-Wertes ("free swell capacity") von superabsorbierendem Polyacrylat-Pulver. Nach WSP 240.2 (05) wird die freie Aufnahmekapazität für eine 0,9 Gewichtsprozent Kochsalzlösung bestimmt. Im Zusammenhang mit der vorliegenden Erfindung wird die Bestimmung des w-Wertes von superabsorbierendem Material, bei dem es sich nicht um Polyacrylat-Pulver handelt, auf entsprechende Weise durchgeführt.

Das superabsorbierende Polymer kann in Form von Fasern oder bevorzugt Partikeln vorliegen.

Bevorzugt ist oder umfasst das superabsorbierende Polymer Polyacrylat. Unter Polyacrylat wird im Rahmen der vorliegenden Erfindung ein synthetisches Polymer verstanden, das als Monomer Acrylsäure (2-Propensäure, CH₂=CH-CO₂H) und/ oder ein Salz hiervon enthält. Der Monomeranteil beträgt insbesondere mehr als 70, z.B. mehr als 80 oder 95 Gewichtsprozent Acrylsäure und/ oder eines Salzes hiervon (bezogen auf das Gesamtgewicht des Polyacrylats). Das Polyacrylat kann als Homopolymer, Copolymer oder Blockpolymer vorliegen.

Um in der Wundauflage einen für die Wundbehandlung vorteilhaften pH-Wert im Bereich von etwa pH 4,0 bis pH 8,5, insbesondere zwischen pH 5,0 und 6,0, zu erreichen liegt das Polyacrylat vorzugsweise als partiell neutralisiertes Polymer vor, insbesondere sollte der Neutralisierungsgrad zwischen 20 % und 90 %, besonders bevorzugt zwischen 45 % und 80% , liegen.

Weiterhin hat es sich gezeigt, dass für die Wundheilung besonders vorteilhafte Effekte auftreten können, wenn der Saugkörper ein Gemisch von Polyacrylatpartikeln umfasst, wobei das Partikelgemisch Polyacrylatpartikel unterschiedlicher Größe enthält, **dadurch gekennzeichnet, dass** das Partikelgemisch
a) 5 bis 100 Gew.-%, vorzugsweise 5 bis 98 Gew.-%, Partikel mit einer Partikelgröße x mit x < 300 µm, und
b) 0 bis 95 Gew.-%, vorzugsweise 2 bis 95 Gew.-%, Partikel mit einer Partikelgröße x mit x > 300 µm,
enthält.

Hinsichtlich weiterer für die vorliegende Erfindung in besonders vorteilhafter Weise verwendbarer Gemische von Polyacrylatpartikeln wird auf die in der WO2009/068249 offenbarten Partikelgemische verwiesen.

Die Partikelgröße wird im Zusammenhang mit der vorliegenden Erfindung analog EDANA 420.2-02 bestimmt, wobei die Siebe (Durchmesser 200 mm) den Angaben entsprechende Lochgrößen aufweisen. Darüber hinaus können auch Siebe mit anderen Lochgrößen wie z.B. 125 µm, 160 µm, 630 µm, 900 µm, und 1500 µm verwendet werden. Hierbei werden trockene Polyacrylatpartikel mit einem Feuchtigkeitsgehalt von weniger als 10 Gew.-% Wasser bezogen auf das Gesamtgewicht der Partikel zugrunde gelegt, wobei der Feuchtigkeitsgehalt gemäß EDANA 450.2-02 bestimmt wird.

Der Saugkörper kann ferner ein Vlies umfassen. Das Vlies kann als Trägermaterial für das quellfähige Polymer dienen.

Bei dem Vlies handelt es sich bevorzugt um ein hydrophiles Fasermaterial. Hierbei können die Fasern des hydrophilen Fasermaterials beispielsweise wasserunlösliche Fasern aus Cellulose, z.B. weitgehend delignifizierte technische Zellstofffasern, z.B. Holzzellstofffasern, bevorzugt mit einer Faserlänge von < 5 mm sein bzw. umfassen. Das Vlies kann auch hydrophiles Fasermaterial aus regenerierter Cellulose, Carboxymethylcellulose, Carboxyethylcellulose, Hydroxymethylcellulose oder Hydroxyethylcellulose enthalten bzw. aus diesem bestehen. Es kann auch auf einem Gemisch von Fasern aus Cellulose, regenerierter Cellulose, Carboxymethylcellulose, Carboxyethylcellulose, Hydroxymethylcellulose oder Hydroxyethylcellulose und thermoplastischen Fasern, beispielsweise aus Polyethylen, Polypropylen oder Polyester, basieren bzw. aus diesem bestehen.

Das quellfähige bzw. superabsorbierende Polymer ist bevorzugt in Form von Partikeln in dem Vlies verteilt.

In einer besonders bevorzugten Ausführungsform umfasst der Saugkörper mindestens ein superabsorbierendes Polyacrylat sowie einen Vlies, der ein Gemisch aus Cellulosefasern und Polypropylenfasern umfasst beziehungsweise auf einer solchen Mischung basiert. Das Vlies kann als Trägermaterial dienen. Die Fasern können in einem so genannten Airlaid-Verfahren zusammen mit Partikeln oder Fasern des superabsorbierenden Polymers zu einer Schicht verarbeitet sein.

In einer besonders bevorzugten Ausführungsform handelt es sich bei dem Saugkörper um ein umhülltes Airlaid, welches ein superabsorbierendes Polyacrylat, Cellulose- und Polypropylenfasern umfasst.

In einer bevorzugten Ausführungsform ist der Saugkörper mit einer Lösung beaufschlagt. Die Beaufschlagung erfolgt vorzugsweise bis zur Sättigung und bevorzugt mit einer wässrigen Lösung, welche das quellfähige bzw. superabsorbierende Material quellen und in einen gelartigen Zustand übergehen lässt. Die wässrige Lösung ist vorzugsweise salzhaltig.

Der Saugkörper kann beispielsweise mit mindestens 600 Gewichtsprozent, mindestens 800 Gewichtsprozent oder mindestens 1000 Gewichtsprozent einer wässrigen Lösung beaufschlagt sein, bezogen auf das Gewicht des trockenen Saugkörpers. Weiterhin ist der Saugkörper vorzugsweise mit weniger als 5000 Gewichtsprozent, z.B. weniger als 3500 Gewichtsprozent, z.B. weniger als 2500 Gewichtsprozent einer wässrigen Lösung beaufschlagt.

In einer besonders bevorzugten Ausführungsform enthält der Saugkörper diejenige Menge einer wässrigen Aktivierungslösung, die seiner maximalen Aufnahmekapazität für Ringerlösung entspricht. Die maximale Aufnahmekapazität für Ringerlösung kann entsprechend der vorgenannten Testmethode WSP 240.2 (05) bestimmt werden, wobei jedoch a) anstelle der in WSP 240.2 (05) verwendeten Kochsalzlösung Ringerlösung eingesetzt wird und b) anstelle der in einer Hülle versiegelten Testsubstanz ("Bag" gemäß Absatz 6.1 von WSP 240.2 (05)) der erfindungsgemäße Saugkörper eingesetzt wird. Die maximale Aufnahmekapazität entspricht der nach diesem Verfahren gravimetrisch ermittelten Gewichtsdifferenz zwischen trockenem Saugkörper und aktiviertem Saugkörper, wobei eine Abweichung der Gewichtsdifferenz um 15 % nach oben oder unten umfasst ist.

Die wässrige Lösung ist eine synthetische Lösung und umfasst keine Körperflüssigkeiten bzw. vom Körper abgesonderte Flüssigkeiten.

Bevorzugt enthält die wässrige Lösung mehr als 50, z.B. mehr als 70, mehr als 80, mehr als 90 oder 100 Volumenprozent Wasser. Sie kann mindestens 5 mmol/l Natrium-Ionen, mindestens 0,1 mmol/l Kalium-Ionen, mindestens 0,1 mmol/l Calcium-Ionen und/oder mindestens 5 mmol/l Chlorid-Ionen enthalten. Optional enthält die wässrige Lösung weitere anorganische Kationen und/oder Anionen, gegebenenfalls organische Anionen, sowie gegebenenfalls Zusätze bioorganischer Verbindungen. Der pH-Wert beträgt vorzugsweise 4 bis 9. Die Viskosität bei 20°C beträgt vorzugsweise zwischen 0,8 mPa_{·}s und 150 mPa·s. Die Viskosität der Lösung wird mit einem Brookfield Viskosimeter bestimmt (Einheit: 1 Pa·s= 1 Ns/m²).

Bevorzugt handelt es sich bei der wässrigen Lösung um eine Ringerlösung. Unter einer Ringerlösung wird eine annähernd zum Blut iso-osmotische synthetische Lösung verstanden, welche Natriumchlorid, Kaliumchlorid und Calciumchlorid in destilliertem Wasser gelöst umfasst. Vorzugsweise enthält die Ringerlösung 147 mmol/l Natrium Ionen, 4,0 mmol/l Kalium Ionen, 3,0 mmol/l Calcium Ionen und 157 mmol/l Chlorid Ionen, wobei eine Abweichung der jeweiligen Ionenkonzentration vom angebenden Wert um 5 % möglich ist.

Ein Vorteil des mit Lösung beaufschlagten Saugkörpers besteht darin, dass unmittelbar nach Beginn der Therapie die Wunde in einem feuchten Milieu gehalten wird und nicht austrocknet, was die Wundheilung unterstützt.

In einer weiteren Ausführungsform kann der Saugkörper, beispielsweise alternativ oder zusätzlich zur oben beschriebenen wässrigen Lösung, eine antimikrobielle Substanz umfassen. Bevorzugt ist oder umfasst die antimikrobielle Substanz eine bei pH-Werten von 4 - 7,5 in kationischer Form vorliegende Substanz mit antimikrobieller Wirkung, beispielsweise Substanzen mit Amino- oder Iminogruppen. Bei der kationischen antimikrobiellen Substanz kann es sich um antimikrobiell wirksame Metallkationen handeln, insbesondere um Silberkationen, beispielsweise um einen Komplex von 1-Vinyl-2-Pyrrolidon mit Silber-Kationen. Besonders geeignete kationische Substanzen mit antimikrobieller Wirkung sind Biguanid-Derivate wie Chlorhexidin oder Polybiguanide, wie Polyethylene Biguanid (PEB), Polytetramethylenbiguanid (PTMB) oder Polyethylenhexamethylenbiguanide (PEHMB). Eine besonders bevorzugtes Polybiguanid ist Polyhexamethylenbiguanid (PHMB, bzw. Polyhexanid). Weitere geeignete kationischen Substanzen mit antimikrobieller Wirkung sind Polyguanidine wie zum Beispiel Polyhexamethylenguanidine (PHMG), N-Octyl-1-[10-(4-Octyliminopyridin- 1-yl)decyl]pyridin-4-imin (Octenedin), quartäre Ammoniumverbindungen, wie zum Beispiel Benzalkoniumchlorid oder Cetylpyridiniumchlorid, Triazine wie zum Beispiel 1-(3-Chloroallyl)-3,5,7-Triaza-1-Azoniaadamantan-Chlorid oder die Ammoniumverbindung Taurolidin.

Neben dem vorstehend beschriebenen Saugkörper kann die erfindungsgemäße Wundauflage zudem eine Wundkontaktschicht und eine verdunstungshemmende Rückschicht enthalten. In einer bevorzugten Ausführungsform umfasst die Wundauflage daher
i) atraumatisch wirkende Wundkontaktschicht,
ii) Saugkörper, und
iii) bevorzugt verdunstungshemmende Folienschicht auf der wundabgewandten Seite.

Bei der Wundkontaktschicht (i) handelt es sich bevorzugt um eine partiell und/oder strukturiert aufgebrachte, atraumatisch wirkende Beschichtung. Die Beschichtung kann direkt auf den Saugkörper aufgebracht werden. Bevorzugt wird sie auf einen Saugkörper aufgebracht, der mit vorstehend beschriebener textiler Hülle umgeben ist. Die Beschichtung ist vorzugsweise eine Silikonbeschichtung, wobei die Beschichtung z.B. porös und von einer Mehrzahl von verhältnismässig dünnen Streifen oder Linien oder inselförmigen Bereichen gebildet sein kann, die durch nicht beschichtete Bereiche voneinander separiert sein können. In diesen nicht beschichteten Bereichen kann die wundzugewandte Seite des Saugkörpers zur Wunde exponiert sein. Die atraumatisch wirkende Beschichtung kann somit einen Überstand bilden, wodurch einerseits ein Verkleben des Saugkörpers mit Wundgewebe verhindert werden kann und andererseits ein gewisser geringer Abstand zwischen der wundzugewandten Seite des Saugkörpers und dem Wundgewebe beibehalten werden kann. Somit kann das poröse Hüllschichtmaterial dreidimensional offen bleiben und einen geringeren Widerstand für den Flüssigkeitsdurchtritt in beiden Richtungen über die Gebrauchsdauer der Wundauflage bereitstellen oder behalten.

Die wundabgewandte Seite des Saugkörpers kann von einer Folie, bevorzugt einer Kunststofffolie, bedeckt sein. Die Folie ist bevorzugt im Wesentlichen keim- und wasserdicht und bietet somit einen verbesserten Verdunstungsschutz.

Die Wundauflage kann auf eine Wunde aufgebracht oder auch zum Austamponieren tiefer Wunden verwendet werden.

Die vorstehenden Erläuterungen zu bevorzugten Ausführungsformen der Wundauflage beziehen sich auf alle Aspekte der vorliegenden Erfindung, d.h. auf das erfindungsgemäße Erzeugnis, auf die erfindungsgemäße Wundauflage, auf die erfindungsgemäße Verwendung und das erfindungsgemäße Verfahren.

Die vorliegende Erfindung ist insbesondere vorteilhaft, wenn es sich bei den Wunden um Brandwunden, um durch mechanische Traumatisierung entstandene Wunden, um eine durch Einwirkung von Chemikalien entstandene Wunde, um eine durch eine Stoffwechselstörung verursachte Wunde, um durch eine Durchblutungsstörung verursachte Wunde, oder um eine durch ein Druckgeschwür verursachte Wunde handelt.

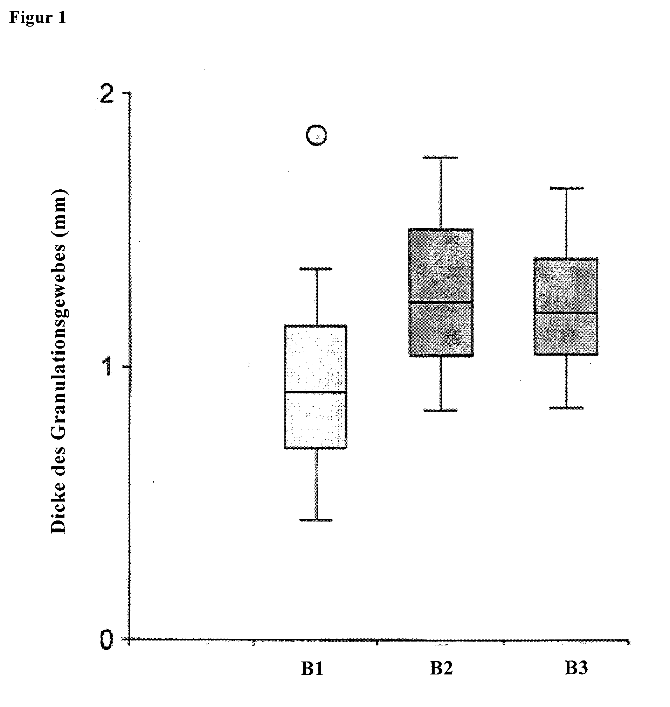

Die folgenden Beispiele illustrieren zusammen mit den Figuren 1 bis 4 die Vorteile der vorliegenden Erfindung.

### BEISPIELE

### Beispiel 1: Verbesserung der Wundheilung

Schweinen wurden im Bereich des dorsalen Adiposegewebes 8 dermoepidermale Wunden pro Versuch mit einem Durchmesser von etwa 3,5 cm zugefügt. Eine erste Versuchsreihe, die im Folgenden als Behandlung 1 bezeichnet wird, umfasste 8 Tage reine Unterdrucktherapie, eine zweite Versuchsreihe, Behandlung 2, umfasste einen ersten Therapieabschnitt mit 4 Tagen Unterdrucktherapie, unmittelbar gefolgt von einem zweiten Therapieabschnitt mit 4 Tagen Therapie in feuchtem bzw. feuchtnassem Milieu. Eine dritte Versuchsreihe, Behandlung 3, umfasste einen ersten Therapieabschnitt mit 6 Tagen Unterdrucktherapie, unmittelbar gefolgt von einem zweiten Therapieabschnitt mit 2 Tagen Therapie in feuchtem bzw. feuchtnassem Milieu. Für den zweiten Therapieabschnitt wurde als Wundauflage das Produkt TenderWet^{®} Plus (Paul Hartmann AG) verwendet, für die Unterdrucktherapie wurden Polyurethanfolie (Hydrofilm^{®}, Paul Hartmann AG) als Abdeckmaterial (a) und offenzelliger Schaumstoff als Wundauflage (c) (Vivanomed^{®}, Paul Hartmann AG) zusammen mit handelsüblichem Adapter, Schlauch (Mittel für die Fluidkommunikation b) und Vakuumgerät eingesetzt.
**Figur 1** zeigt, dass sich bei den erfindungsgemäßen Behandlungen B2 (4 Tage Unterdruck + 4 Tage feuchtes Milieu) und B3 (6 Tage Unterdruck + 2 Tage feuchtes Milieu) im Vergleich zu einer Unterdrucktherapie B1 gleicher Dauer (8 Tage) die Bildung von Granulationsgewebe erheblich verbessert hat.
**Figur 2** zeigt, dass sich bei den erfindungsgemäßen Behandlungen B2 (4 Tage Unterdruck + 4 Tage feuchtes Milieu) und B3 (6 Tage Unterdruck + 2 Tage feuchtes Milieu) im Vergleich zu einer Unterdrucktherapie B1 gleicher Dauer (8 Tage) die Neo-Vascularization verbessert hat. Es konnte eine erhebliche Verbesserung der Stimulation der Gefäßneubildung erreicht werden.
**Figur 3** zeigt, dass sich bei den erfindungsgemäßen Behandlungen B2 (4 Tage Unterdruck + 4 Tage feuchtes Milieu) und B3 (6 Tage Unterdruck + 2 Tage feuchtes Milieu) im Vergleich zu einer Unterdrucktherapie B 1 gleicher Dauer (8 Tage) die Bildung von Fibroblasten verbessert hat. Es konnte eine erhebliche Erhöhung der Fibroblastendichte erreicht werden.

Die in den Figuren 1 bis 3 gezeigten positiven Effekte des erfindungsgemäßen Therapieschemas waren für den Fachmann nicht zu erwarten.

### Beispiel 2: Scavenging von Entzündungsmediatoren durch Polyacrylat-haltige Wundauflagen

In Wunden mit Heilungsstörungen oder problematischer Heilung sind eine Reihe von Proteinen beschrieben worden, die die Heilung direkt oder indirekt behindern. Dazu zählen DAMPs und PAMPs aber auch Matrix-Metalloproteinasen und andere Proteine. Folglich sollen diese Mediatoren bevorzugt der Wunde - besonders bei hyperinflammatorischen Zuständen - entzogen werden. Im erfindungsgemäßen Behandlungsschema kann dieser Entzug vorteilhaft stattfinden, wie nachfolgender Versuch zeigt:

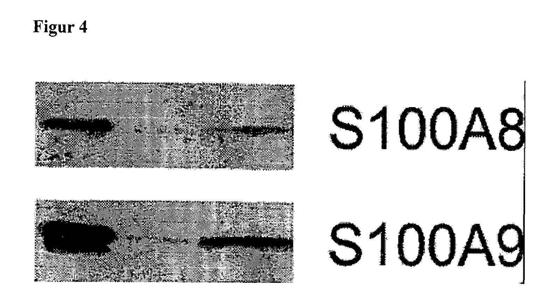

Wundexsudat wurde von chronischen Wunden gewonnen (Eming et al., 2008). Die Menge, die 50 Microgramm Gesamtprotein entspricht, wurde mit 100 mg FAVOR^{®} PAC 300 Superabsorber (mit Ringerlösung zur Sättigung vorgequollen) für 2 Stunden inkubiert. Danach wurde die Flüssigkeit von dem Superabsorber getrennt, dieser 2-fach mit einem Überschuss an Ringerlösung gewaschen und die gebundenen Proteine mit SDS-Probenpuffer (10 mg SDS gelöst in 0,25 mL 0,5 M Tris/HCl, pH 6,8, 0,115 mL Glyzerin (87 %), 0,5 mL H₂O und 1-2 Tropfen Bromphenolblau (1 % in Wasser)) gelöst. Es erfolgte die Auftrennung der Proteine nach ihrem Molekulargewicht in einem 10 % Polyacrylamidgel (Eckert und Kartenbeck: Proteine: Standardmethoden der Molekular- und Zellbiologie: Präparation, Gelelektrophorese, Membrantransfer und Immundetektion. Springer, Berlin, 1997, ISBN-10: 3540612785). Nach Transfer auf eine PVDF Membran und immunologischer Detektion mit spezifischen, affinitätsgereinigten Antikörpern (Calgranulin A Antibody (C-10), sc-48352; Calgranulin B Antibody (C-19), sc-8114; Santa Cruz Biotechnology, Inc., Bergheimer Str. 89-2, 69115 Heidelberg, Deutschland) entsprechend der Vorschriften des Herstellers ergab sich folgendes Bild, siehe Figur 4:
- in der linken Spur ist die Wundflüssigkeit vor der Inkubation aufgetragen;
- in der mittleren Spur ist Wundflüssigkeit nach Inkubation aufgetragen;
- in der rechten Spur ist das an dem FAVOR^{®} PAC 300 Superabsorber gebundene Protein aufgetragen.

Nach Immundetektion sind die entsprechenden Banden S100A8 und S100A9 klar zu erkennen und die Menge an gebundenem S100A8 und S100A9 Protein zeigt eine klare Bindung an das Polyacrylat-Polymer an. Der Entzug der Mediatoren ist somit ein unerwarteter Effekt, der zum klinischen Erfolg des erfindungsgemäßen Behandlungsschemas beitragen kann.

## Patentansprüche

1. Erzeugnis, zur Anwendung in der therapeutischen Behandlung von Wunden am menschlichen oder tierischen Körper, wobei das Erzeugnis eine Vorrichtung zur Unterdrucktherapie von Wunden umfasst, **dadurch gekennzeichnet, dass** die therapeutische Behandlung einen ersten Therapieabschnitt umfasst, in welchem Unterdrucktherapie mittels der Vorrichtung durchgeführt wird, und einen darauf folgenden zweiten Therapieabschnitt umfasst, in welchem die therapeutische Behandlung unter Einsatz einer Wundauflage ohne Erzeugung eines Unterdrucks durchgeführt wird, wobei die Wundauflage einen Saugkörper umfasst, welcher ein quellfähiges Polymer, insbesondere ein superabsorbierendes Polymer, umfasst.

2. Erzeugnis, zur Anwendung in der therapeutischen Behandlung von Wunden am menschlichen oder tierischen Körper, wobei das Erzeugnis eine Wundauflage umfasst, und wobei die Wundauflage einen Saugkörper umfasst, der ein quellfähiges Polymer, insbesondere ein superabsorbierendes Polymer, umfasst, **dadurch gekennzeichnet, dass** die therapeutische Behandlung einen ersten Therapieabschnitt umfasst, in welchem Unterdrucktherapie mittels einer Vorrichtung zur Unterdrucktherapie von Wunden durchgeführt wird, und einen darauf folgenden zweiten Therapieabschnitt umfasst, in welchem die therapeutische Behandlung unter Einsatz der Wundauflage ohne Erzeugung eines Unterdrucks durchgeführt wird.

3. Erzeugnis, zur Anwendung in der therapeutischen Behandlung von Wunden am menschlichen oder tierischen Körper, wobei das Erzeugnis eine Vorrichtung zur Unterdrucktherapie von Wunden und eine Wundauflage umfasst, und wobei die Wundauflage einen Saugkörper umfasst, der ein quellfähiges Polymer, insbesondere ein superabsorbierendes Polymer, umfasst, **dadurch gekennzeichnet, dass** die therapeutische Behandlung einen ersten Therapieabschnitt umfasst, in welchem Unterdrucktherapie mittels der Vorrichtung durchgeführt wird, und einen darauf folgenden zweiten Therapieabschnitt umfasst, in welchem die therapeutische Behandlung unter Einsatz der Wundauflage ohne Erzeugung eines Unterdrucks durchgeführt wird.

4. Erzeugnis gemäß einem der vorstehenden Ansprüche, wobei der erste Therapieabschnitt mit Abklingen oder Abschluss der inflammatorischen Phase endet.

5. Erzeugnis gemäß einem der vorstehenden Ansprüche, wobei der erste Therapieabschnitt sich über einen Zeitraum von 1 bis 8 Tagen erstreckt.

6. Erzeugnis gemäß einem der vorstehenden Ansprüche, wobei der zweite Therapieabschnitt während oder unmittelbar nach der inflammatorischen Phase beginnt.

7. Erzeugnis gemäß einem der vorstehenden Ansprüche, wobei der zweite Therapieabschnitt sich über einen Zeitraum von 1 bis 8 Tagen erstreckt.

8. Erzeugnis gemäß einem der vorstehenden Ansprüche, wobei der Saugkörper eine flüssigkeitsdurchlässige, textile Hülle und darin angeordnet ein superabsorbierendes Polymer umfasst.

9. Erzeugnis gemäß einem der vorstehenden Ansprüche, wobei die Wundauflage umfasst:
i) atraumatisch wirkende Wundkontaktschicht,
ii) Saugkörper, und
iii) bevorzugt verdunstungshemmende Folienschicht auf der wundabgewandten Seite.

10. Erzeugnis gemäß einem der vorstehenden Ansprüche, wobei der Saugkörper ein Vlies umfasst, welches bevorzugt Cellulosefasern enthält.

11. Erzeugnis gemäß einem der vorstehenden Ansprüche, wobei der Saugkörper mit einer Lösung, bevorzugt Ringerlösung, beaufschlagt ist.

12. Erzeugnis gemäß einem der vorstehenden Ansprüche, wobei der Saugkörper eine antimikrobielle Substanz umfasst.

13. Erzeugnis gemäß einem der vorstehenden Ansprüche, wobei die Vorrichtung zur Durchführung der Wundtherapie mittels Unterdruck umfasst
(a) ein luftundurchlässiges Abdeckmaterial zum luftdichten Verschließen von Wunde und Wundumgebung, wobei ein Wundraum gebildet wird,
(b) ein Mittel zum Bereitstellen von Fluidkommunikation zwischen dem Wundraum und einer außerhalb des Abdeckmaterials befindlichen Unterdruckquelle, so dass ein Unterdruck im Wundraum herstellbar ist, und Flüssigkeiten aus dem Wundraum absaugbar sind,
(c) eine Wundauflage, welche eine im Wesentlichen gleichmäßige Verteilung des Unterdrucks im Wundraum ermöglicht, bevorzugt eine Wundauflage, die offenzelligen Schaumstoff, insbesondere offenzelligen Polyurethanschaumstoff, enthält.

14. Kombination aus Vorrichtung zur Unterdrucktherapie von Wunden und einer Wundauflage, zur Anwendung in der therapeutischen Behandlung von Wunden am menschlichen oder tierischen Körper, wobei die Wundauflage einen Saugkörper umfasst, der ein quellfähiges Polymer, insbesondere ein superabsorbierendes Polymer, umfasst, **dadurch gekennzeichnet, dass** die therapeutische Behandlung einen ersten Therapieabschnitt umfasst, in welchem Unterdrucktherapie mittels der Vorrichtung durchgeführt wird, und einen darauf folgenden zweiten Therapieabschnitt umfasst, in welchem die therapeutische Behandlung unter Einsatz der Wundauflage ohne Erzeugung eines Unterdrucks durchgeführt wird.

15. Verwendung einer Wundauflage in der therapeutischen Behandlung von Wunden am menschlichen oder tierischen Körper, wobei die Wundauflage einen Saugkörper umfasst, der ein quellfähiges Polymer, insbesondere ein superabsorbierendes Polymer, umfasst, **dadurch gekennzeichnet, dass** die therapeutische Behandlung einen ersten Therapieabschnitt umfasst, in welchem Unterdrucktherapie mittels einer Vorrichtung zur Unterdrucktherapie von Wunden durchgeführt wird, und dass die therapeutische Behandlung einen darauf folgenden zweiten Therapieabschnitt umfasst, welcher unter Einsatz der Wundauflage ohne Erzeugung eines Unterdrucks durchgeführt wird.
